# EUROPÄISCHE PATENTSCHRIFT

(11) **EP 0 782 853 B1**
(45) Veröffentlichungstag und Bekanntmachung des Hinweises auf die Patenterteilung: **11.06.2003**
(21) Anmeldenummer: 96108536.2
(22) Anmeldetag: 29.05.1996
(51) Int. Cl.: A61K 9/52, C12N 11/04, A61K 9/00

(54) **Bioaktive Kapsel mit veränderlicher Hülle**
Bioactive capsule with modifiable shell
Capsule bioactive avec une paroi modifiable

(30) Priorität: 31.05.1995 DE 19519804
(43) Veröffentlichungstag der Anmeldung: 09.07.1997
(73) Patentinhaber: SCHREZENMEIR, Jürgen, D-55128 Mainz (DE); Pommersheim, Rainer, 76185 Karlsruhe (DE); Vogt, Walter, Dr., 65207 Wiesbaden (DE)
(72) Erfinder: SCHREZENMEIR, Jürgen, D-55128 Mainz (DE); Pommersheim, Rainer, 76185 Karlsruhe (DE); Vogt, Walter, Dr., 65207 Wiesbaden (DE)
(74) Vertreter: Pöhner, Wilfried Anton, Dr.

(56) Entgegenhaltungen:
- WO-A-93/16687
- DE-A- 4 312 970
- DATABASE WPI Section Ch, Week 8538 Derwent Publications Ltd., London, GB; Class A23, AN 85-234403 XP002032364 & JP 60 153 905 A (AGENCY OF IND SCI & TECHNOLOGY) , 13.August 1985

## Beschreibung

Die Erfindung bezieht sich auf ein Verfahren zur Strukturänderung bioaktiver Kapsel, insbesondere zum Einsatz in lebendes Gewebe oder bei biotechnologischen Anwendungen, mit einem lebende Zellen und/oder Enzyme enthaltenden Kern und einer Hülle, die aus mehreren, den Kern jeweils vollständig umschließenden Einzelschichten aufgebaut ist, die aus einem porösen Netzwerk miteinander verflochtener Makromoleküle bestehen und die maximale Durchlaßweite der Poren größer oder gleich der größten, zur Versorgung der im Kern enthaltenen Zellen und/oder den Enzymen zugeführten und/oder von ihnen produzierten Moleküle ist.

Bioaktive Kapseln werden eingesetzt, um lebendes oder lebloses biologisches Material, speziell Zellen und Enzyme, zu immobilisieren, ihre Funktion außerhalb der natürlichen Umgebung langfristig aufrecht zu erhalten und ihre Handhabung zu erleichtern. Derartige Notwendigkeiten bestehen beispielsweise beim Einsatz des Materials in das Gewebe von Lebewesen, etwa zur Übernahme der Funktion der Langerhans'schen Inseln bei Diabetikern, oder im Rahmen biotechnologischer Anwendungen. Die bioaktive Kapsel ist mit einer Hülle versehen, die das im Kern enthaltene Material einschließt und stabilisiert sowie als semipermeable Membran wirkt. Während Stoffe, die im Kern umgesetzt werden oder zur Aufrechterhaltung seiner Funktion notwendig sind, die Hülle möglichst ungehindert passieren sollen, müssen die aggressiven Komponenten in einem Bioreaktor oder eines Immunsystems, wie Abwehrzellen oder Antikörper, vom Kern ferngehalten werden, damit er nicht vollständig zerstört oder in seiner Funktion zumindest beeinträchtigt wird.

Über eine vorgegebene mechanische Stabilität und Durchlässigkeit hinaus besteht eine Vielzahl weiterer Anforderungen an die Hülle der Kapsel. Ihr Herstellungsverfahren hat so schonend abzulaufen, daß das eingeschlossene Material nicht geschädigt wird. Weiterhin muß das Hüllenmaterial sowohl mit den eingeschlossenen Zellen oder Enzymen als auch, insbesondere bei der Implantation in lebendiges Gewebe, mit der Umgebung verträglich sein und darf durch sie nicht angegriffen werden. Die verschiedenen Aufgaben lassen sich vorteilhaft mit einer bioaktiven Kapsel erfüllen, deren Hülle mehrere Einzelschichten umfaßt, die jeweils aus einem Netzwerk miteinander verflochtener Makromoleküle aufgebaut sind (DE-OS 43 12 970). Die einzelnen Schichten optimieren die Funktion der Hülle jeweils im Hinblick auf eine einzelne oder wenige Anforderungen, d.h. die mechanische Stabilität, die Gewebeverträglichkeit oder die Durchlässigkeit der Hülle werden durch unterschiedliche Schichten erzielt.Die Publikation Database WP I Section Ch., Week 8538, Derwent Publications Ltd, London, offenbart einschichtige, flüssigkeitsdurchlässige Membrane aus Polyaminosäuren mit spiralförmiger Struktur, die aufgebrochen werden können. Die WO 93/16687 A offenbart eine Vielzahl von Materialien zur Herstellung einschaliger Kapseln zur Umhüllung biologisch aktiven Materials, wobei u.a. auch Hyaluronicsäure Erwähnung findet.

Im Stande der Technik besteht jedoch der Nachteil, daß die Eigenschaften der Kapselhülle bei ihrer Produktion vorgegeben werden und mit Ausnahme einer vollständigen Zerstörung im Verlaufe des Einsatzes nicht veränderbar sind. Vielfach besteht jedoch der Anspruch, einen Stoff auf Vorrat zu produzieren und ihn nach Bedarf, etwa bei einem bestimmten Produktionsschritt, freizusetzen. Diese Möglichkeit ist bei den bekannten bioaktiven Kapseln nicht gegeben.

Vor diesem Hintergrund hat sich die Erfindung zur Aufgabe gestellt, ein Verfahren zur Strukturänderung bioaktiver Kapsel mit einer mehrschichtigen Hülle anzugeben, deren Permeabilität im Verlauf des Einsatzes gezielt veränderbar ist.

Diese Aufgabe wird erfindungsgemäß dadurch gelöst, daß mindestens eine der Schichten aus einem Material besteht, das als Funktion einer Ionenkonzentration und/oder physikalischer Größen und/oder durch Reagenzien die Struktur verändert oder auflöst, daß infolge der Strukturänderung Poren, die den Kern der Kapsel mit ihrer Umgebung verbinden, geöffnet, geschlossen oder in ihrem Durchmesser verändert werden.

Der lebende Zellen und/oder Enzyme enthaltende Kern der vorgeschlagenen bioaktiven Kapsel ist von einer Hülle umgeben, deren Einzelschichten aus einem porösen Netzwerk miteinander verflochtener Makromoleküle aufgebaut sind. Mindestens eine der Schichten ist vollständig auflösbar oder in ihrer Struktur veränderlich, indem physikalische Größen oder die Kapsel umgebende Konzentrationen von Ionen oder nicht ionischen Reagenzien variiert werden. Dabei ist es möglich, auch im Kapselinneren angeordnete Schichten chemisch in ihrer Struktur zu verändern, wenn die beeinflussenden Ionen oder Moleküle so klein sind, daß sie in die poröse Hülle eindringen. Infolge der Strukturänderung werden durchgehende Poren, die den Kern der Kapsel mit ihrer Umgebung verbinden, geöffnet, geschlossen oder in ihrem Durchmesser verändert. Durch die gezielte Beeinflussung der Porenstruktur läßt sich das Ausschütten im Inneren der Kapsel produzierter Stoffe oder das Eindringen von Substanzen in den Kern steuern. Speziell im letzteren Fall kann die Verbindung auch zwischen dem Kern und einer äußeren, spezielle Substanzen enthaltenden Hülle hergestellt werden, ohne daß Poren zwischen dem Kern und der äußeren Umgebung der Kapsel in ihrem engsten Durchmesser verändert werden. Um die Funktionsfähigkeit der bioaktiven Kapsel langfristig sicherzustellen, ist es nötig, daß die Durchlaßweite der Poren, die den Kern mit der äußeren Umgebung der Kapsel verbinden, größer oder gleich der größten, zur Versorgung der im Kern enthaltenen Zellen benötigten und/oder den Enzymen zugeführten Molekülen einstellbar ist. Ebenso muß der maximale Porendurchmesser die Abgabe aller produzierten Moleküle gestatten.

Die veränderliche Hüllenstruktur erweitert die Einsatzbereiche der erfindungsgemäßen bioaktiven Kapsel im Vergleich zu herkömmlichen Kapseln wesentlich, wobei alle bisherigen Einsatzmöglichkeiten erhalten bleiben. Insbesondere erlaubt es die mehrschichtige Struktur der Hülle weiterhin, die Eigenschaften auf gestellten Anforderungen flexibel einzustellen. Die Veränderbarkeit der Struktur ermöglicht eine Anpassung der Funktion der Kapsel an Veränderungen in dem Milieu, in dem sie arbeitet.

Je nach verwendeter veränderlicher Schicht erfolgt die Funktionsanpassung durch einen aktiven Eingriff des Anwenders, d.h. es wird z.B. ein die Schichtstruktur beeinflussender Stoff in das Medium abgegeben, in dem sich die Kapsel befindet, oder die Schichtstruktur paßt sich selbsttätig an die Erfordernisse an. Ein Beispiel für den letzteren Fall ist eine Kapsel, deren Hüllstruktur durch einen von ihr verarbeiteten oder mit diesem durch eine chemische Gleichgewichtsreaktion in Verbindung stehenden Stoff verändert wird. Die Kapsel kann nicht nur in ihrem Inneren produzierte Substanzen zu einem willkürlich wählbaren Zeitpunkt in die Umgebung abgeben, sondern ihr auch bestimmte Substanzen entziehen oder sie in andere Stoffe umwandeln. Somit zeichnet sich die vorgeschlagene bioaktive Kapsel dadurch aus, daß sie die Möglichkeit eines aktiven Eingriffs in in ihrem Inneren oder ihrer Umgebung ablaufende Reaktionen eröffnet oder selbsttätig zu ihrer Steuerung in der Lage ist.

In einer bevorzugten Ausgestaltung der bioaktiven Kapsel enthält ihre Hülle Polyelektrolytkomplexe. Sie gestatten den Aufbau biologisch gut verträglicher, leicht herzustellender, stabiler Schichten mit sehr geringen Dicken und einem einstellbaren Porendurchmesser, wobei Kombinationen von Polyanionen, etwa Polyacrylsäure, Polykationen, z.B. Polyethylenimin, und/oder neutralen Polymeren möglich sind. Da die Änderung der Porenweite einer Polyelektrolytschicht mit Veränderungen weiterer Eigenschaften, beispielsweise des Quellungsgrades, d.h. des Anteils in der Schicht enthaltenen Wassers, oder der mechanischen Festigkeit, einhergeht, bestehen in diesem Fall zusätzliche Beeinflussungsmöglichkeiten für die Hüllenstruktur.

Zweckmäßig ist die Beeinflussung der Struktur der veränderlichen Schicht durch die Konzentration eines gelösten Salzes in der Kapselumgebung. Dies gilt im speziellen, wenn am Hüllenaufbau Polyelektrolytkomplexe beteiligt sind, da bei der durch Coulombkräfte vermittelten Komplexbildung die Ladungen der ionischen Gruppen an den makromolekularen Ketten im Wettbewerb mit denen der Salzionen stehen. Infolge dessen läßt sich die Bildung der Polyelektrolytkomplexe durch Salze in ausreichender Konzentration verhindern oder rückgängig machen und damit die Schichtstruktur verändern. Der besondere Vorteil besteht darin, daß jeder Komplex spezifisch auf bestimmte Salzkonzentrationen und -arten in seiner Umgebung reagiert, so daß bei Schichten aus verschiedenen Polyelektrolytkomplexen gezielte Salzzugaben oder -entfernungen die Permeabilität bestimmter Schichten verändern. Einen ähnlichen Einfluß wie die Salzionen haben Hydroxid- und Hydroniumionen, so daß auch der pH-Wert der Umgebungslösung zur Beeinflussung der Schichtstruktur geeignet ist.

Physikalische Größen, durch die die Struktur einer Schicht beeinflußbar ist, sind insbesondere die Temperatur oder der Lichteinfall. Beispielsweise zeigen Polymere mit Azogruppen Konformationsänderungen bei Lichteinfall, die die Durchlässigkeit der Kapselmembran verändern und zu einer einfachen und direkten Steuerung der Kapsel nutzbar sind. Ebenso lassen sich Gestaltänderungen der Polymermoleküle infolge von Temperaturvariationen zur Beeinflussung der Hüllenstruktur verwenden, wobei z.B. Makromoleküle mit Polyisopropylacrylamidgruppen die Möglichkeit einer Durchlässigkeitsänderung der Hülle bei Temperaturvariationen im physiologischen Bereich eröffnet, die im Hinblick auf die zu beeinflussenden biologischen Prozesse besonders vorteilhaft ist.

Ist die Änderung der Schichtstruktur reversibel, läßt sich die Wirkung der bioaktiven Kapsel über längere Zeit hinweg und wiederholt steuern. Bei entsprechender Wahl der Schichtbestandteile lassen sich mit allen oben besprochenen Steuerparametern reversible Strukturveränderungen erreichen.

Darüber hinaus ist vorgesehen, daß die Struktur einer Schicht durch Enzyme veränderlich ist, wobei die Veränderung auch in ihrem vollständigen Abbau bestehen kann. Als Beispiel sei hier der Abbau einer Schicht aus Hyaluronsäure-Polykationkomplexen durch Hyaluronidase genannt. Auch auf diese Weise ist eine einfache Veränderung der Hülldurchlässigkeit erzielbar, die jedoch in der Regel irreversibel ist.

Die unterschiedlichen Möglichkeiten der Schichtbeeinflussung sind einzeln oder auch in beliebigen Kombinationen einsetzbar, so daß eine große Vielfalt von Kontrollmöglichkeiten für die bioaktive Kapsel vorhanden ist.

Je nach den an sie gestellten Anforderungen weist die Kapsel bevorzugt eine kugel- oder plattenförmige Gestalt auf. Während eine Kugelgestalt, die sich speziell bei Mikrokapseln bewährt hat, die Oberfläche der Kapsel minimiert, wird sie durch eine plattenförmige Gestalt, die vorzugsweise für Kapseln mit größerem Kernvolumen geeignet ist, maximiert. Letzteres ist z.B. dann von Vorteil, wenn die Kapseln bei einer biotechnologischen Anwendung auf einer Trägeroberfläche angeordnet sind und dem Arbeitsmedium eine möglichst große Oberfläche darbieten sollen.

Alternativ wird vorgeschlagen, daß die Kapsel die Gestalt eines Hohlzylinders aufweist, wobei der Kern eine Schicht der Zylinderwandung darstellt, die sowohl in Richtung auf die Zylinderachse als auch außenseitig durch Hüllschichten umschlossen ist. Im Sinne der Erfindung sind auch einem Hohlzylinder topologisch äquivalente Gestalten möglich, beispielsweise eine gekrümmte, hohle Faser. Derartige Kapselgestalten gestatten es, insbesondere wenn die Hüllen zur Zylinderachse und -außenseite hin unterschiedliche Porenweiten aufweisen, daß die Lösungen, durch die dem Kern Stoffe zugeführt werden und die von ihm produzierte Stoffe aufnehmen, vollständig voneinander getrennt sind. Auf diese Weise lassen sich Reinigungsprozesse vermeiden oder die Funktion von Organen, wie der Niere, nachbilden. Ferner können dem Kern auf der Außen- und Innenseite des Zylinders Medien zur Verarbeitung zugeführt werden, die miteinander unverträglich sind.

Enthält der Kern Substanzen zur Beeinflussung oder Aufrechterhaltung der in ihm ablaufenden Reaktionen, so ist die Kapsel auch in einem Milieu einsetzbar, in dem die im Kern eingeschlossenen Zellen oder Enzyme nicht arbeiten könnten. Beispielsweise kann durch eine entsprechende Eisenkonzentration im Kern sichergestellt werden, daß auf Eisen angewiesene Mikroorganismen dieses selbst dann zur Verfügung gestellt bekommen, wenn sich die Kapsel in einer Umgebung befindet, die eisenbindende Substanzen beinhaltet.

Ein zusätzlicher Vorzug der erfindungsgemäßen bioaktiven Kapsel besteht darin, daß sie sich dazu verwenden läßt, Substanzen durch Bereiche zu transportieren, die die Substanz in unerwünschter Weise beeinflussen oder durch sie beeinflußt werden. Zu diesem Zweck wird zunächst die Hüllendurchlässigkeit der Kapsel derart eingestellt, daß die Substanz in den Kern gelangt, und anschließend so weit vermindert, daß sie im Kapselkern eingeschlossen ist und keine unerwünschten Stoffe dorthin gelangen. In dieser Form wird die Kapsel zum Bestimmungsort gebracht, wobei infolge des verringerten Porendurchmessers gewährleistet ist, daß die transportierten Substanzen das umgebende Medium nicht verunreinigen oder hierdurch zerstören. Am Bestimmungsort wird die Hülldurchlässigkeit durch Erweiterung der Poren erhöht, so daß die Substanz aus dem Kern ausgeschüttet wird.

Weitere Einzelheiten, Merkmale und Vorteile der Erfindung lassen sich dem nachfolgenden Beschreibungsteil entnehmen, in dem anhand der Zeichnung ein Ausführungsbeispiel der Erfindung näher erläutert wird. Die Zeichnung zeigt in prinzipienhafter Darstellung:
- Figur 1: Schnitt durch eine erfindungsgemäße bioaktive Kapsel
- Figur 2: Funktionsweise der den Kern einhüllenden Schichten

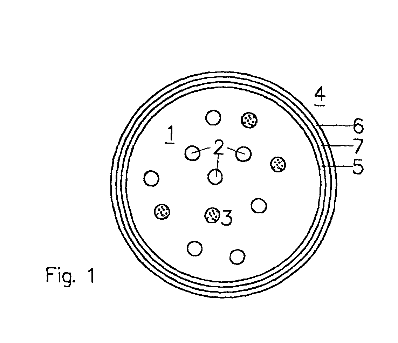

Der Kern (1) der in Figur 1 dargestellten bioaktiven Kapsel enthält als aktive Komponenten Zellen und/oder Enzyme (2) sowie Nährstoffe (3), die im umgebenden Medium des Reaktors nicht vorhanden sind oder nicht durch die Hülle (1) in den Kern eindringen können, aber zur Aufrechterhaltung der in ihr ablaufenden Reaktionen notwendig sind. Die Hülle besteht aus mehreren Schichten, die den Kern (1) vollständig umschließen. Durch diese Mehrschichtigkeit ist eine flexible Anpassung an die jeweiligen Erfordernisse erzielbar, wobei die innere Schicht (5) mit den im Kern enthaltenen Zellen und/oder Enzymen (2) verträglich ist, während die äußere Schicht (6) an das umgebende Medium (4) angepaßt ist. Zwischen beiden befindet sich eine in ihrer Struktur veränderliche Schicht (7).

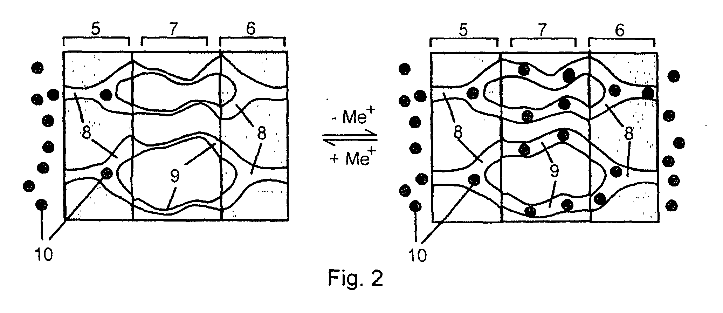

Die Wirkungsweise der veränderlichen Schicht zeigt Figur 2. Im Gegensatz zur inneren Schicht (5) und äußeren Schicht (6), deren Poren (8) einen festen Durchmesser aufweisen, sind die Durchmesser der Poren (9) in der veränderlichen Schicht (7) variabel. Dabei erfolgt die Beeinflussung des Porendurchmesser durch Zugabe oder Entfernen von Metallionen (Me). Die Variation des Porendurchmessers hat zur Folge, daß im Kern (1) produzierte Moleküle (10) nur dann durch die Poren (8, 9) der Hülle in die Umgebung (4) des Reaktors gelangen können, wenn die Konzentration der Metallionen (Me) so geartet ist, daß der Durchmesser der Poren (9) hinreichend groß ist. Auf diese Weise läßt sich durch Veränderung der Konzentration der Metallionen (Me) eine gezielte Ausschüttung der Moleküle (10) aus der bioaktiven Kapsel erreichen.

## Patentansprüche

1. Verfahren zur Strukturänderung bioaktiver Kapsel, insbesondere zum Einsatz in lebendes Gewebe oder bei biotechnologischen Anwendungen, mit einem lebende Zellen und/oder Enzyme enthaltenden Kern und einer Hülle, die aus mehreren, den Kern jeweils vollständig umschließenden Einzelschichten aufgebaut ist, die aus einem porösen Netzwerk miteinander verflochtener Makromoleküle bestehen,
und die maximale Durchlaßweite der Poren (8, 9) größer oder gleich der größten, zur Versorgung der im Kern enthaltenen Zellen und/oder den Enzymen zugeführten und/oder von ihnen produzierten Moleküle (11) ist.
**dadurch gekennzeichnet, daß**
- mindestens eine der Schichten (7) aus einem Material besteht, das als Funktion einer Ionenkonzentration und/oder physikalischer Größen und/oder durch Reagenzien die Struktur verändert oder auflöst,
- infolge der Strukturänderung Poren (8, 9), die den Kern der Kapsel mit ihrer Umgebung verbinden, geöffnet, geschlossen oder in ihrem Durchmesser verändert werden.

2. Verfahren nach Anspruch 1, **dadurch gekennzeichnet, daß** die Schichten (5, 6, 7) der Hülle Polyelektrolytkomplexe enthalten.

3. Verfahren nach Anspruch 2, **dadurch gekennzeichnet, daß** die Schichten (5, 6, 7) Polyacrylanionen und/oder Polymethacrylanionen und/oder Schwefelsäureester polymerer Kohlenhydrate und/oder Polyethylenimin und/oder Polydimethyldiallylammonium und/oder Chitosan beinhalten.

4. Verfahren nach Anspruch 1 bis 3, **dadurch gekennzeichnet, daß** die Struktur einer Schicht (7) durch die Konzentration eines gelösten Salzes in der Kapselumgebung beeinflußbar ist.

5. Verfahren nach einem der vorhergehenden Ansprüche, **dadurch gekennzeichnet, daß** die Struktur einer Schicht (7) durch den pH-Wert in der Kapselumgebung beeinflußbar ist.

6. Verfahren nach einem der vorhergehenden Ansprüche, **dadurch gekennzeichnet, daß** die Struktur einer Schicht (7) von ihrer Temperatur abhängig ist.

7. Verfahren nach Anspruch 6, **dadurch gekennzeichnet, daß** die Schicht (7) Polymere mit Polyisopropylacrylamidgruppen enthält.

8. Verfahren nach einem der vorhergehenden Ansprüche, **dadurch gekennzeichnet, daß** die Struktur einer Schicht (7) durch Lichteinfall beeinflußbar ist.

9. Verfahren nach Anspruch 7, **dadurch gekennzeichnet, daß** die Schicht (7) Polymere mit Azogruppen beinhaltet.

10. Verfahren nach einem der vorhergehenden Ansprüche, **dadurch gekennzeichnet, daß** die Strukturänderung reversibel ist.

11. Verfahren nach einem der vorhergehenden Ansprüche, **dadurch gekennzeichnet, daß** die Struktur einer Schicht (7) durch Enzyme veränderlich ist.

12. Verfahren nach Anspruch 11, **dadurch gekennzeichnet, daß** die Schicht (7) Hyaluronsäure- oder Xylankomplexe enthält.

13. Verfahren nach einem der vorhergehenden Ansprüche, **dadurch gekennzeichnet, daß** sie eine kugeloder plattenförmige Gestalt besitzt.

14. Verfahren nach einem der Ansprüche 1 bis 12, **dadurch gekennzeichnet, daß** sie die Gestalt eines Hohlzylinders aufweist, wobei der Kern (1) eine Schicht der Zylinderwandung darstellt, die sowohl in Richtung auf die Zylinderachse als auch außenseitig durch Hüllschichten umschlossen ist.

15. Verfahren nach einem der vorhergehenden Ansprüche, **dadurch gekennzeichnet, daß** der Kern (1) Substanzen zur Beeinflussung oder Aufrechterhaltung der in ihm ablaufenden Reaktionen enthält.

16. Verfahren nach einem der Ansprüche 10 bis 15 für den Transport von Substanzen durch Medien, die die Substanz in unerwünschter Weise beeinflussen oder durch sie beeinflußt werden, **gekennzeichnet durch** folgende Verfahrensschritte:
- Herstellen eines Kontaktes zwischen Kapseloberfläche und zu transportierender Substanz,
- Einstellung der Hüllendurchlässigkeit derart, daß die Substanz in den Kapselkern gelangt,
- Vermindern der Hüllendurchlässigkeit derart, daß die Substanz im Kapselkern eingeschlossen ist und keine unerwünschten Stoffe in den Kern gelangen,
- Transport der Kapsel zum Bestimmungsort,
- Erhöhung der Hüllendurchlässigkeit derart, daß die Substanz aus dem Kern gelangt.

## Claims

1. Method for the structural modification of bioactive capsules, in particular for use in living tissue or in biotechnological applications, comprising a nucleus containing living cells and/or enzymes and an envelope, which is constructed of a plurality of individual layers which in each case enclose the nucleus completely and consist of a porous network of entangled macromolecules, and the maximum permeation width of the pores (8, 9) is greater than or equal to the biggest molecules (11) that are administered for supplying the cells and/or the enzyme and/or are produced thereby **characterised in that**
- at least one of the layers consists of a material. that, as a function of an ion concentration and/or physical parameters and/or by means of reagents, modifies or dissolves the structure,
- by virtue of the structural modification, pores, which connect the nucleus of the capsule to its environment are opened, closed or changed in their diameter.

2. Method according to claim 1, **characterised in that** the layers (5, 6, 7) of the envelope comprise polyelectrolyte complexes.

3. Method according to claim 2, **characterised in that** the layers (5, 6, 7) contain polyacrylic anions and/or polymethacrylic anions and/or sulphuric acid esters of polymeric carbohydrates and/or polyethyleneimine and/or polydimethyldiallylammonium and/or chitosan.

4. Method according to claims 1 to 3, **characterised in that** the structure of a layer (7) can be influenced by the concentration of a dissolved salt in the capsule environment.

5. Method according to one of the preceding claims, **characterised in that** the structure of a layer (7) can be influenced by the pH in the capsule environment.

6. Method according to one of the preceding claims, **characterised in that** the structure of a layer (7) is dependent on its temperature.

7. Method according to one of the preceding claims, **characterised in that** the layer (7) contains polymers with polyisopropylacrylamide groups.

8. Method according to one of the preceding claims, **characterised in that** the structure of a layer (7) can be influenced by light incidence.

9. Method according to claim 7, **characterised in that** the layer (7) includes polymers with azo groups.

10. Method according to one of the preceding claims, **characterised in that** the structural change is reversible.

11. Method according to one of the preceding claims, **characterised in that** the structure of a layer (7) can be modified by means of enzymes.

12. Method according to claim 11, **characterised in that** the layer (7) contains hyaluronic acid or xylane complexes.

13. Method according to one of the preceding claims, **characterised in that** it has a spherical or platelet shape.

14. Method according to one of claims 1 to 12, **characterised in that** it has the shape of a hollow cylinder, the nucleus (1) representing a layer of the cylinder wall, which is enclosed by envelope layers both towards the cylinder axis and outwardly.

15. Method according to one of the preceding claims, **characterised in that** the nucleus (1) contains substances for influencing or sustaining the reactions taking place therein.

16. Method according to one of claims 10 to 15 for the transport of substances through media which influence the substance in a desirable manner or are influenced by it, **characterised by** the following steps:
- production of contact between the capsule surface and the substance to be transported,
- adjustment of the envelope permeability such that the substance passes into the capsule nucleus
- reduction of the envelope permeability such that the substance is enclosed in the capsule nucleus and no undesirable substance passes into the nucleus,
- transport of the capsule to the place of use,
- increase of the envelope permeability such that the substances passes out of the nucleus.

## Revendications

1. Procédé permettant de modifier la structure de capsules bioactives, notamment dans des tissus vivants ou dans des applications biotechnologiques, composées d'un noyau renfermant des cellules vivantes et/ou des enzymes et d'une enveloppe constituée de plusieurs couches entourant complètement le noyau, ces couches étant elles-mêmes composées d'un réseau poreux de molécules entrelacées et la largeur maximale de passage des pores (8, 9) étant supérieure ou égale à la plus grande molécule apportée pour alimenter les cellules et/ou les enzymes contenues dans le noyau et/ou à la plus grande molécule produite par celles-ci (11),
**caractérisé en ce que**
- au moins l'une des couches (7) de l'enveloppe se compose d'une substance modifiant ou dissolvant la structure en fonction de la concentration en ions et/ou de grandeurs physiques et/ou de réactifs,
- cette modification de structure entraînant l'ouverture, la fermeture ou la variation du diamètre des pores (8, 9) qui assurent la liaison entre le noyau de la capsule et l'environnement de cette dernière

2. Procédé selon la revendication 1, **caractérisé en ce que** les couches (5, 6, 7) de l'enveloppe renferment des complexes de polyélectrolytes.

3. Procédé selon la revendication 2, **caractérisé en ce que** les couches (5, 6, 7) renferment des anions polyacryliques et/ou polyméthacryliques et/ou des esters d'acide sulfurique d'hydrates de carbone polymères et/ou du polyéthylénimine et/ou du polydiméthyldiallylammonium et/ou du chitosan.

4. Procédé selon les revendications 1 à 3, **caractérisé en ce que** l'on peut influencer la structure d'une couche (7) en agissant sur la concentration d'un sel dissout dans le milieu environnant la capsule.

5. Procédé selon l'une des revendications précédentes, **caractérisé en ce que** l'on peut influencer la structure d'une couche (7) en agissant sur le pH du milieu environnant la capsule.

6. Procédé selon l'une des revendications précédentes, **caractérisé en ce que** la structure d'une couche (7) dépend de sa température.

7. Procédé selon la revendication 6, **caractérisé en ce que** la couche (7) contient des polymères avec des groupements de polyisopropylacrylamide.

8. Procédé selon l'une des revendications précédentes, **caractérisé en ce que** l'on peut influencer la structure d'une couche (7) en modifiant l'incidence de la lumière.

9. Procédé selon la revendication 6, **caractérisé en ce que** la couche (7) contient des polymères avec des groupements azo.

10. Procédé selon l'une des revendications précédentes, **caractérisé en ce que** la modification de la structure est réversible.

11. Procédé selon l'une des revendications précédentes, **caractérisé en ce que** l'on peut influencer la structure d'une couche (7) par des enzymes.

12. Procédé selon l'une des revendications précédentes, **caractérisé en ce que** la couche (7) contient des complexes d'acide hyaluronique ou de xylane.

13. Procédé selon l'une des revendications précédentes, **caractérisé en ce que** la capsule a une forme sphérique ou plate.

14. Procédé selon l'une des revendications 1 à 12, **caractérisé en ce que** la capsule a la forme d'un cylindre creux, le noyau (1) formant une couche de la paroi du cylindre entourée par les couches de l'enveloppe d'une part, en direction de l'axe du cylindre et d'autre part, vers l'extérieur.

15. Procédé selon l'une des revendications précédentes, **caractérisé en ce que** le noyau (1) contient des substances influençant ou maintenant les réactions qui s'y déroulent.

16. Procédé selon l'une des revendications 10 à 15 pour le transport de substances dans des fluides ayant des effets indésirables sur ces substances ou influencés par ces substances,
**caractérisé par les étapes suivantes :**
- réalisation d'un contact entre la surface de la capsule et la substance à transporter,
- réglage de la perméabilité de l'enveloppe de manière à ce que la substance pénètre dans le noyau de la capsule,
- réduction de la perméabilité de l'enveloppe de sorte que la substance soit enfermée dans le noyau de la capsule et qu'aucune substance indésirable ne puisse pénétrer dans le noyau,
- transport de la capsule jusqu'à son lieu de destination,
- augmentation de la perméabilité de l'enveloppe pour permettre à la substance de sortir du noyau.
